Europäisches Patentamt

European Patent Office

Office européen des brevets

(19)

(11) Publication number: **0 138 101 B1**

# EUROPEAN PATENT SPECIFICATION

(45) Date of publication of patent specification: **10.04.91**

(51) Int. Cl.⁵: **C12N 15/30**, C12P 21/02, A61K 39/005, G01N 33/569

(21) Application number: **84111343.4**

(22) Date of filing: **24.09.84**

(54) Genetically engineered organisms expressing surface proteins of T. Cruzi.

(30) Priority: **30.09.83 US 537798**

(43) Date of publication of application:
**24.04.85 Bulletin 85/17**

(45) Publication of the grant of the patent:
**10.04.91 Bulletin 91/15**

(84) Designated Contracting States:
**DE FR GB**

(56) References cited:
**US-A- 3 911 097**
**US-A- 3 993 743**

**PROCEEDINGS OF THE NATIONAL ACADEMY OF SCIENCES OF THE UNITED STATES OF AMERICA, vol. 79, no. 4, February 1982 (Baltimore, US), N. NOGUEIRA et al.:"Specific glycoprotein antigens on the surface of insect and mammalian stages of Trypanosoma cruzi", pp. 1259-1263**

**THE JOURNAL OF EXPERIMENTAL MEDICINE, vol. 153, no. 3, March 1, 1981 (New York), N. NOGUEIRA et al.:"Trypanosoma eruzi-Surface Antigens of Blood and Culture**

Forms", pp. 629-639

(73) Proprietor: **ROCKEFELLER UNIVERSITY**
**1230 York Avenue**
**New York, NY 10021(US)**

(72) Inventor: **Lizardi, Paul**
**500 East 63 Street**
**New York New York 10021(US)**
Inventor: **Nogueira, Nadia**
**6 Greenholm**
**Princeton New Jersey 08540(US)**

(74) Representative: **Hoffmeister, Helmut, Dr. Dipl.-Phys.**
**Patentanwalt Goldstrasse 36**
**W-4400 Münster(DE)**

## Description

This invention concerns genetically engineered organisms which express surface proteins of T. cruzi , the causative agent of Chagas disease. The proteins are immunogenic and are useful in detection of infection and immunoprophylaxis of the disease.

## Background

Two major surface antigens on Trypanosoma cruzi have been identified (Nogueira, N., Chaplan, S., Tydings, J., Unkeless, J. and Cohn, Z., Exp. Med. 153 629 (1981)). One, a Mr 75,000 glycoprotein (GP) is specific for the culture forms (insect-host stages) of the organisms - epimastigotes and metacyclic trypomastigotes. The other, a Mr 90,000 GP is specific for vertebrate - host stages of the organisms (bloodstream - form trypomastigotes). The two GP are unrelated and do not cross react immunogeneically. Because of their specificity, the surface proteins are potential diagnostic markers for Chagas disease.

## Summary

Recombinant plasmids harboring clones of double-stranded cDNA for coding an insect stage specific surface glycoprotein of T. cruzi have been constructed. The bacterial clones express the recombinant protein making it useful as a source of large amounts of antigen for both diagnostic and immunoprophylactic purposes.

This invention especially relates to a plasmid pIF8 as deposited at the ATCC at Rockvolle, Maryland, USA, on September 27, 1983 under No. 400 82.

The invention also relates to said plasmid in a suitable host bacterium, especially Escherichia coli K 12.

The invention further relates to a DNA sequence which expresses the peptide portion of the surface insect stage 75.000 Dalton glycoprotein of Trypanosoma cruzi which consists of the base-pairs being the difference between pIF8 and puCl3. The DNA sequence can have about 600 base-pairs.

It is a further object of the invention to provide a plasmid containing the DNA sequence of claim 4 or 5.

Another object of the invention is to provide a method for producing the peptide portion of the surface insect stage 75.000 Dalton glycoprotein of T. cruzi which comprises expressing a plasmid of claim 1 or claim 6 or a plasmid containing a DNA coding for the same peptide as the plasmids of claims 1 and 6. A more particular object of the present invention is a method of diagnosing Chagas disease in vitro comprising contacting the IgG containing fraction of blood from a potential patient having chagas disease with peptides produced according to claim 8 in an immunological reaction.

## Details

Details on the isolation, and characterization of the Mr 75,000 and Mr 90,000 surface glycoproteins of T. cruzi have been disclosed in the publications by Nogueira et al. J. Exp. Med. 153 629 (1981)) and Nogueira et al. Proc. Nat'l Acad. Sci. USA 79 1259 (1982).

## Availability of Plasmid

The plasmids disclosed in the present invention bear the deposit number PIF8 and are deposited with The Rockefeller University, 1230 York Avenue, New Yor, New York 10021. Preferred plasmids of the present invention are also deposited at the American Type culture Collection, Bethesda, Maryland and bear ATCC deposit number 40082.

## Preparation of plasmids

Poly A$^+$ RNA was isolated from 3 week-old strain epimastigote cultures, purified by oligo dT cellulose chromotography, and translated in vitro in the wheat germ cell-free translation system. Translation products were immunoprecipitated with either the IgG fraction of serum from a patient with chronic Chagas disease or with rabbit antiserum raised against epimastigote surface proteins. The IgG fraction of patients with Chagas' disease recognized 4 major bands of the in vitro translated material, one of which, a 34,000 Mr peptide may be the unglycosilated precursor of 75,000 Mr peptide.

The total poly A$^+$ RNA was converted to cDNA with reverse transcriptase. The double-stranded cDNA was inserted into the Pst I site of the plasmid pUCl3 by G-C tailing and the recombinant plasmids used to transform E. coli JM83. Colonies were selected for ampicillin resistance and initially screened by plating on YT medium supplemented with X-gal. Inserts into the cloning site disrupt the coding of B-galactosidase, resulting in the loss of -complementation and enzymatic activity. Thus, plasmids with inserts gave rise to colonies that cannot hydrolyze X-gal and are consequently white. Plasmids without inserts or with small non-disruptive inserts gave rise to blue colonies. 1303 white, ampicillin-resistant colonies were selected for further screening by in situ radioimmunoassay.

Selection of Specific Plasmids

Bacterial cells were transferred to nitrocellulose filters, grown overnight, and lysed in situ . The filters were then incubated with the patient serum, washed extensively, and stained with $I^{125}$ -labelled protein A from S. aureus . Eight colonies gave a strong positive signal upon re-screening with the patient serum. None of the eight, however, reacted with anti-75k monoclonal antibodies or with rabbit antiserum anti-75K.

Analysis of plasmid DNA from these eight clones showed inserts ranging in size from 100 base pairs to 600 base pairs. Thus, even short insert sequences can produce antigenic fragments recognized by polyvalent serum.

Because of its unusual culture morphology, one clone, pIT8, was chosen for further study. Unlike the other immunoreactive clones or its JM83 parent, plf8 grows in liquid culture as a rough strain which suggests that the trypanosome polypeptide may be expressed on the bacterial cell surface.

pIF8 contains a pUCl3 plasmid with a cDNA insert of 600 base pairs. To prove its trypanosome origin, the $^{32}$P-labelled insert was hybridized with trypanosome DNA in a Southern blot. The cloned DNA hybridized to several fragments generated by Hind II, Pst I, Sal I, or BamH I digestion of total DNA isolated form epimastigotes. These restriction enzymes do not cut the 600 basepair insert itself. This multiple banding pattern could result from the presence of intervening sequences or multiple gene copies.

The $^{32}$P-labelled insert was also used as the probe in a Northern blot of epimastigote RNA. The probe hybridized to an RNA of approximately 1000 nucleotides in both total RNA and polyA$^+$ RNA preparations. It also hybridized to a second species of approximately 2100 nucleotides in the total RNA. In comparison, there is very little hybridization in a Northern blot of total RNA or polyA$^+$ RNA isolated from vertebrate-stage parasites (tryptomastigotes). The 2100 nucleotide species is also present in the total tryptomastigote RNA. The results of these blot experiments strongly suggest stage-specific mRNA processing.

The difference in size between the 1000 nucleotide mRNA and the 600 base-pair cDNA insert indicates that the pIF8 clone must not code for a full-length gene. However, since the patient serum is able to react with the bacteria, the clone must encode and express at least one epitope. Nogueira has shown that all Chagas' disease patient sera tested recognize the same trypanosome antigens. Therefore, the pIF8 clone is likely to be useful in the diagnosis of the chronic illness.

Immunological detection of clones containing T, cruzi cDNA which expresses surface proteins

After lysis and washing, the filters were incubated for two hours at room temperature with the IgG fraction of Chagas disease patient serum diluted 1/300 into 50mM tris-HCl/150mM NaCL/3% BSA/1% NP-40/0.2% SDS. The serum had been pre-adsorbed with lysate prepared from the parental bacterial string JM83. The filters were then washed extensively with several changes of tris-saline and incubated for one-hour at room temperature with $I^{125}$ labelled protein A (specific activity approximately 5 x $10^6$ cpm/ug) diluted to $10^6$ cpm/ml in the same incubation buffer. The filters were washed extensively in the tris-saline buffer containing NP-40 and SDS. Protein A was labelled using the iodogen technique. Total epimastigote lysate served as a positive control on all filters.

This interaction between clone and lung antibodies against the surface protein of T. cruzi in human serum forms an essential basis for a diagnostic method for Chagas disease. These cloned peptides or their synthetic counterparts may be used in this method.

Northern blot analysis of RNA from epimastigotes and

The RNA samples were electrophoresed on a 1M formaldehyde-1% agarose slab gel and blotted overnight onto BA83 nitrocellulose paper (Schleicher and Schuell).

The filter paper was pre-hybridized for 4 hours at 42° C in 50 % formamide, 5XSSC, 4X Denhardt's buffer, 0.1% SDS, and 10% dextran sulfate. It was then hybridized for 12 hours in this same solution containing the $^{32}$P-labelled pIF8 probe and 100 ug/ml E. coli DNA. The filter was washed in 2xSSPE - 0.1% SDS for 30 minutes at 37° C and then in 0.1 XSSPE - 0.1% SDS at 37° C for 30 minutes.

## Claims

1. Plasmid pIF8 as deposited at the ATCC at Rockville, Maryland, USA, on September 27, 1983, under No. 400 82.

2. Plasmid of claim 1 in a suitable host bacterium.

3. Plasmid of claim 2, whereby said host bacterium is Escherichia coli K 12.

4. DNA sequence coding for the peptide portion of the surface insect stage 75.000 Dalton glycoprotein of Trypanosoma cruzi and consisting of the base-pairs being the difference between plF8 and puCl3.

5. DNA sequence of claim characterized in having about 600 base pairs.

6. Plasmid containing the DNA sequence of claim 4 or 5.

7. Method for producing the peptide portion of the surface insect stage 75.000 Dalton glycoprotein of T. cruzi which comprises expressing a plasmid of claim 1 or claim 6 or a plasmid containing a DNA coding for the same peptide as the plasmids of claims 1 and 6.

8. Method of diagnosing Chagas disease in vitro comprising contacting the IgG containing fraction of blood from a potential patient having chagas disease with peptides produced according to claim 7 in an immunological reaction.

**Revendications**

1. Plasmide p1F8 tel que déposé à l'ATCC à Rockville, Maryland, USA, le 27 Septembre 1983, sous le N° 400 82.

2. Plasmide de la revendication 1 dans une bactérie hôte appropriée.

3. Plasmide de la revendication 2, dans lequel cette bactérie hôte est Escherichia coli K 12.

4. Séquence d'ADN codant pour la partie peptidique de la glycoprotéine de surface de 75 000 Daltons du stade insecte de Trypanosoma cruzi et constituée des paires de base représentant la différence entre p1F8 et puC13.

5. Séquence d'ADN de la revendication 4, caractérisée en ce qu'elle a environ 600 paires de base.

6. Plasmide contenant la séquence d'ADN des revendications 4 ou 5.

7. Procédé de préparation de la partie peptidique de la glycoprotéine de surface de 75 000 Daltons du stade insecte de T. cruzi, qui comprend l'expression d'un plasmide de la revendication 1 ou de la revendication 6 ou d'un plasmide contenant un ADN codant pour le même peptide que les plasmides des revendications 1 et 6.

8. Méthode de diagnostic de la maladie de chagas in vitro, comprenant la mise en contact de la fraction contenant l'IgG du sang d'un malade potentiel atteint de la maladie Chagas avec des peptides préparés conformément à la revendication 7 dans une réaction immunologique.

**Ansprüche**

1. Plasmid plF8, wie hinterlegt im ATCC, Rockville, Maryland, USA, am 27. September 1983, unter der Nummer 400 82.

2. Plasmid nach Anspruch 1 in einem geeigneten Wirtsbakterium.

3. Plasmid nach Anspruch 2, wobei das Wirtsbakterium Escherichia coli, K 12, ist.

4. DNA-Sequenz mit der Codierung für den Peptid-Abschnitt des 75 000-Dalton-Oberflächen-Glycoproteins des Insektenstadiums von Trypanosoma Cruzi und bestehend aus den Basenpaaren, die die Differenz zwischen plF8 und puCl3 darstellen.

5. DNA-Sequenz nach Anspruch 4, dadurch gekennzeichnet, daß es ungefähr 600 Basenpaare besitzt.

6. Plasmid enthaltend die DNA-Sequenz des Anspruches 4 oder 5.

7. Verfahren zur Herstellung des Peptid-Abschnittes des 75.000-Dalton-Oberflächen-Glycoproteins des Insektenstadiums von T. Cruzi, wobei das Verfahren die Produktion eines Plasmids des Anspruches 1 oder Anspruches 6 oder eines Plasmids, das eine DNA enthält mit der Codierung für das gleiche Peptid wie die Plasmide des Anspruches 1 und 6, umfaßt.

8. Verfahren zur in-vitro Diagnose der Chagas-Krankheit, dadurch gekennzeichnet, daß die IgG enthaltende Fraktion des Blutes eines Patienten, der möglicherweise Chagas-Krankheit hat, in einer immunologischen Reaktion mit Peptiden kontaktiert wird, die nach Anspruch 7

hergestellt wurden.